(19) Europäisches Patentamt
European Patent Office
Office européen des brevets

(11) Publication number: **0 055 583**
**B1**

(12) **EUROPEAN PATENT SPECIFICATION**

(45) Date of publication of patent specification: **28.08.85**

(21) Application number: **81306025.8**

(22) Date of filing: **22.12.81**

(51) Int. Cl.[4]: **C 07 D 239/95,**
C 07 D 403/12,
C 07 D 403/14,
A 61 K 31/495, A 61 K 31/53
// (C07D403/12, 239:95,
251:46),(C07D403/12, 239:95,
251:54),(C07D403/12, 239:95,
237:20),(C07D403/12, 239:95,
237:22),(C07D403/12, 239:95,
241:20),(C07D403/14, 239:95,
217:24, 239:48)

(54) **Antihypertensives.**

(30) Priority: **29.12.80 GB 8041411**

(43) Date of publication of application:
**07.07.82 Bulletin 82/27**

(45) Publication of the grant of the patent:
**28.08.85 Bulletin 85/35**

(84) Designated Contracting States:
**AT BE CH DE FR GB IT LI LU NL SE**

(56) References cited:
**GB-A-1 176 854**
**US-A-3 935 213**
**US-A-4 062 844**
**US-A-4 101 548**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LI LU NL SE AT**

(72) Inventor: **Campbell, Simon Fraser**
**Grey Friars Upper Street Kingsdown**
**Deal Kent (GB)**
Inventor: **Plews, Rhona Margret**
**32 Glebelands Ash**
**Canterbury Kent (GB)**

(74) Representative: **Wood, David John**
**Pfizer Limited Ramsgate Road**
**Sandwich Kent CT13 9NJ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 055 583 B1

**Description**

This invention relates to therapeutic agents which are novel derivatives of 4-amino-6,7-dimethoxy-2-piperazinoquinazoline. Such compounds are useful as regulators of the cardiovascular system, and, in particular, the treatment of hypertension.

In US Patent Nos. 4 062 844, 4 101 548, 3 935 213 and GB—A—1 176 854 derivatives of 2-(4'-substituted-piperazin-1-yl-)-4-amino-6,7-dimethoxy-quinoxoline having antihypertensive activity are disclosed.

The novel compounds according to the invention are those having the general formula:

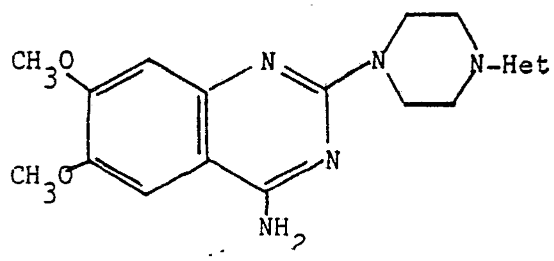

---(I)

and their pharmaceutically acceptable acid addition salts, wherein Het is a nitrogen-containing heterocyclic group selected from:—

(a) 2- or 4-pyrimidinyl unsubstituted or substituted by 1 or 2 substituents each selected from $C_1$—$C_4$ alkyl, $C_1$—$C_4$ alkoxy, halo and —$NR^1R^2$ where $R^1$ is $C_1$—$C_4$ alkyl and $R^2$ is $C_1$—$C_4$ alkyl or $C_3$—$C_7$ cycloalkyl, or $R^1$ and $R^2$ taken together with the nitrogen atom to which they are attached represent morpholino;

(b) 3-pyridazinyl unsubstituted or substituted by 1 substituent selected from $C_1$—$C_4$ alkoxy and phenoxy; and

(c) 1,3,5-triazin-2-yl substituted by 2 substituents selected from $C_1$—$C_4$ alkoxy and —NH.($C_1$—$C_4$ alkyl).

"Halo" means fluoro, chloro, bromo or iodo. It is preferably chloro.

The preferred cycloalkyl group is cyclopentyl.

Alkyl and alkoxy chains of 3 or 4 carbon atoms can be straight or branched chain.

The most preferred individual compounds are those in which "Het" is:

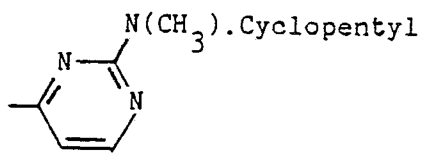

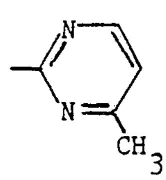,

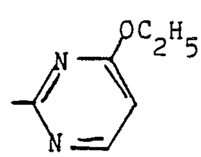,

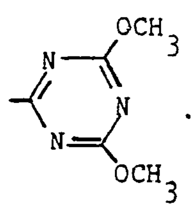,

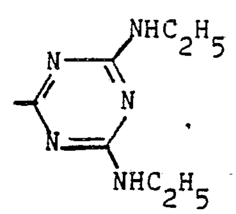,

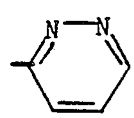

or

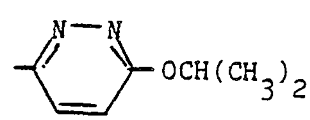.

Pharmaceutically acceptable acid addition salts of the compounds of the invention are those formed from acids which form non-toxic acid addition salts containing pharmaceutically acceptable anions, such as the hydrochloride, hydrobromide, sulphate or bisulphate, phosphate or acid phosphate, acetate, maleate, fumarate, succinate, lactate, tartrate, citrate, gluconate, saccharate, mesylate and p-toluenesulphonate salts.

(1) The compounds of the formula (I) can be prepared by reacting a quinazoline of the formula:

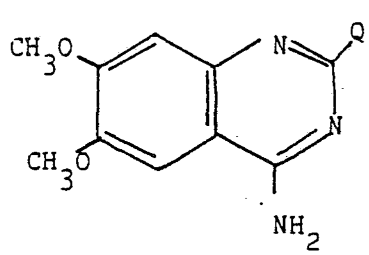

---(III)

where Q is a facile leaving group such as halo, $C_1$—$C_4$ alkoxy or $C_1$—$C_4$ alkylthio, with a piperazine of the formula:—

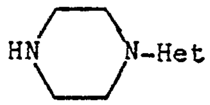

---(IV)

"Q" is preferably Cl.

In a typical procedure the reactants are heated together, e.g., at 70—150°C, preferably under reflux, in a suitable solvent, e.g., *n*-butanol, for up to about 25 hours, the exact period of time of course depending on the nature of the reactants and the temperature employed, as will be known to those skilled in the art. The product can then be isolated and purified by conventional procedures.

If compound (IV) is added in the form of an acid addition salt, then a tertiary amine base such as triethylamine is preferably added.

The starting materials of the formula (IV) are either known compounds or may be prepared by methods analogous to those of the prior art, many of such methods being illustrated in the Preparations hereinafter.

(2) The compounds of the formula (I) can also be prepared by reacting a 2-piperazinoquinazoline of the formula:

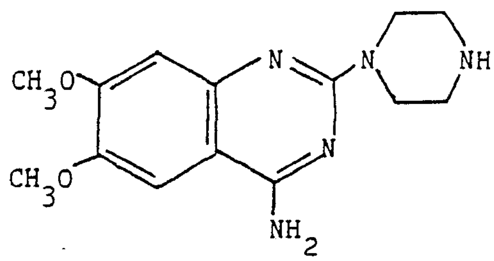

---(V)

with a heterocycle of the formula:

Q—Het (VI)

where Q and Het are as for route (1).

Q is preferably Cl.

The reaction may be carried out in a similar manner to the previous method. Similarly, the product may be isolated and purified by conventional procedures. The starting materials of the formulae (V) and (VI) are either known compounds or may be prepared by methods analogous to those of the prior art.

(3) Some of the compounds of the formula (I) can be prepared from other compounds of the formula (I) For example compounds of the formula (I) in which "Het" is substituted by —$NR^1R^2$ where $R^1$ and $R^2$ are as defined for formula (I) can be prepared by reacting the corresponding compound in which "Het" is substituted by halo (preferably chloro) with the appropriate amine of the formula $R^1R^2NH$. Generally, fairly vigorous reaction conditions are necessary, e.g. heating the reactants in a suitable solvent, e.g. *n*-butanol, at up to 180°C in a bomb, for up to about 48 hours. In addition, a halo substituent on "Het" can be reduced to H by hydrogenation, e.g. by hydrogenation over Pd/C.

The pharmaceutically acceptable acid addition salts of the compounds of the formula (I) can be prepared by conventional procedures, e.g. by reacting the free base with the appropriate acid in an inert organic solvent, and collecting the resulting precipitate of the salt by filtration. If necessary, the product may then be re-crystallized to purify it.

The antihypertensive activity of the compounds of the formula (I) is shown by their ability to lower the blood pressure of conscious spontaneously hypertensive rats and conscious renally hypertensive dogs, when administered orally at doses of up to 5 mg/kg.

The compounds of the invention can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules either alone or in admixture with excipients, or in the form of elixirs or suspensions containing flavouring or colouring agents. They may be injected parenterally, for example, intramuscularly, intravenously or subcutaneously. For parenteral administration, they are best used in the form of a sterile aqueous solution which may contain other solutes, for example, enough salts or glucose to make the solution isotonic.

Thus the invention also provides a pharmaceutical composition comprising a compound of the formula (I) or pharmaceutically acceptable acid addition salt thereof together with a pharmaceutically acceptable diluent or carrier.

The invention further provides a compound of the formula (I), or a pharmaceutically acceptable acid addition salt thereof, for use in treating hypertension in a human being.

The compounds of the invention can be administered to humans for the treatment of hypertension by either the oral or parenteral routes, and may be administered orally at dosage levels approximately within the range 1 to 20 mg/day for an average adult patient (70 kg) given in a single dose or up to 3 divided doses. Intravenous dosage levels would be expected to be about 1/5 to 1/10th of the daily oral dose. Thus for an average adult patient, individual oral doses in tablet or capsule form will be approximately in the range from 1/3 to 20 mg of the active compound. Variations will necessarily occur depending on the weight and condition of the subject being treated and the particular route of administration chosen will be known to those skilled in the art.

The invention is illustrated by the following Examples:

The compounds in Example 1 to 13 were prepared by procedures similar to that of "Method A", which illustrates the preparation of an analogous compound to those claimed. Similarly the compounds of Examples 14 to 20 were prepared similarly to "Method B", which again illustrates the preparation of an analogous compound to those claimed. All temperatures are in °C.

Method "A"

*Preparation of 4-Amino-6,7-dimethoxy-2-[4-(4-phenylpyrimidin-2-yl) piperazino] quinazoline, hemihydrate*

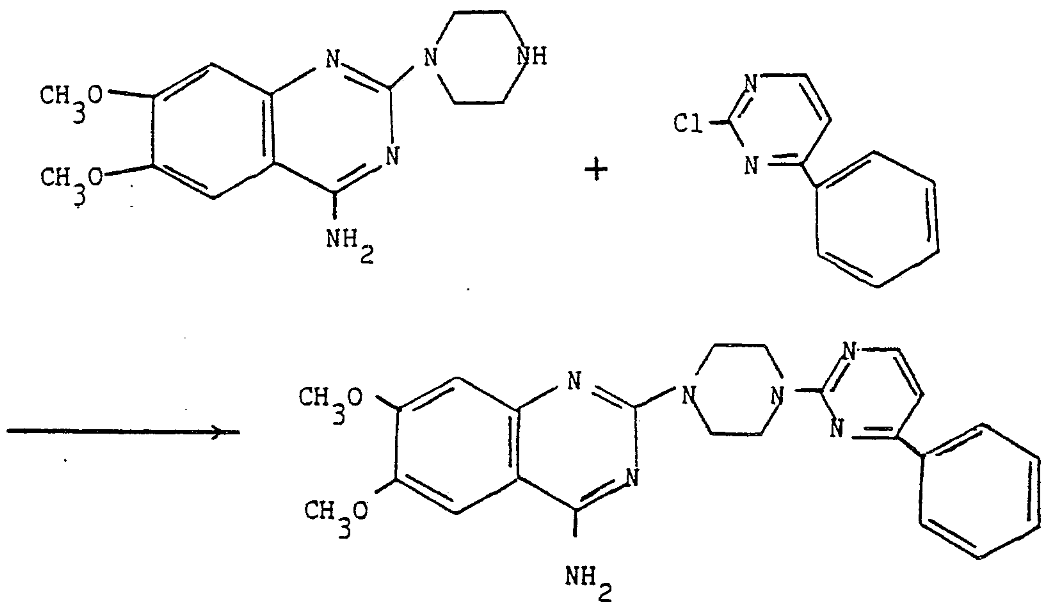

4-Amino-6,7-dimethoxy-2-piperazino-quinazoline (3.44 g) and 2-chloro-4-phenylpyrimidine (2.5 g) (J. Chem. Soc., 1951, 2328) in *n*-butanol (250 ml) were heated under reflux for six hours. After cooling the solid product was collected, washed with diethyl ether, and partitioned between chloroform and saturated aqueous sodium carbonate solution. The chloroform layer was separated, the aqueous layer extracted with chloroform and the combined chloroform layers were washed with water, dried ($Na_2SO_4$) and evaporated *in vacuo*. The residue (5 g) was chromatographed on silica eluting with chloroform and chloroform-methanol (97.5:2.5 by volume). Appropriate fractions were combined, evaporated and the resulting solid crystallized from dimethylformamide (DMF)/diethyl ether to give *4-amino-6,7-dimethoxy-2-[4-(4-phenylpyrimidin-2-yl)piperazino] quinazoline hemihydrate,* (2.28 g), m.p. 250° (dec).

*Analysis %:*

| | | | |
|---|---|---|---|
| Found: | C,63.5; | H,5.8; | N,21.8. |
| Calculated for $C_{24}H_{25}N_7O_2 \cdot \frac{1}{2}H_2O$: | C,63.7; | H,5.8; | N,21.7 |

The following Examples illustrate the invention:

Examples 1 to 13

The following compounds were prepared in the same general manner as Method A, but in some cases the reaction mixture was evaporated *in vacuo* and then purified as described. Other modifications were as follows. In Example 2, the product was collected from the cooled reaction mixture then re-crystallized from ethanol. In Examples 4, 5, and 11, the initial reaction was carried out in ethanol and in Example 5 the product, after chromatography, was converted to the dihydrochloride salt. In Example 7, after basification and extraction, the residue was re-crystallized from methanol. In Example 8, the solid product from the reaction mixture was take up in DMF/diethyl ether and precipitated with water.

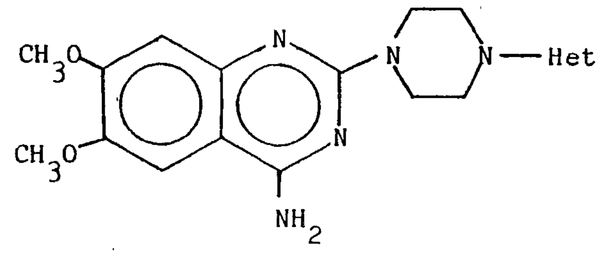

| Example No. | Het | Form Isolated and m.p. | Analysis % (calculated figures in brackets) C | H | N |
|---|---|---|---|---|---|
| 1 | 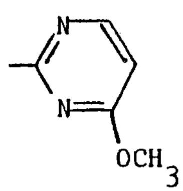 | Free base 202-203° | 57.1 (57.4 | 6.0 5.8 | 25.1 24.7) |
| 2 | 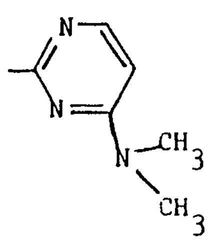 | hydrochloride 3/4 $H_2O$ 262-264° | 52.2 (52.2 | 6.2 6.2 | 24.4 24.3) |

| Example No. | Het | Form Isolated and m.p. | Analysis % (calculated figures in brackets) C | H | N |
|---|---|---|---|---|---|
| 3 | 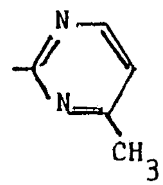 | 1/3 ethanolate<br>225-226° | 59.2<br>(59.5 | 6.2<br>6.4 | 24.9<br>24.7) |
| 4 | 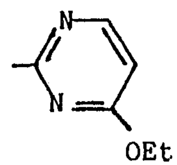 | fee base<br>227° | 58.5<br>(58.4 | 6.4<br>6.1 | 23.4<br>23.8) |
| 5 | 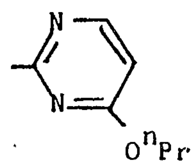 | dihydrochloride<br>hydrate<br>265-269° | 48.9<br>(48.8 | 5.6<br>6.1 | 19.0<br>19.0) |

| Example No. | Het | Form Isolated and m.p. | Analysis % (calculated figures in brackets) C | H | N |
|---|---|---|---|---|---|
| 6 | 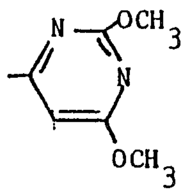 | free base<br>194–195° | 56.0<br>(56.2 | 5.9<br>5.9 | 23.2<br>22.9) |
| 7 | 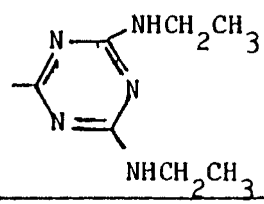 | free base<br>130–133° | 55.1<br>(55.5 | 6.7<br>6.7 | 30.8<br>30.8) |
| 8 | 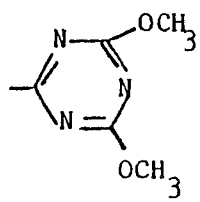 | hydrochloride<br>hydrate<br>225–226° | 46.9<br>(47.3 | 5.7<br>5.6 | 23.1<br>23.2) |

| Example No. | Het | Form Isolated and m.p. | Analysis % (calculated figures in brackets) | | |
|---|---|---|---|---|---|
| | | | C | H | N |
| 9 | 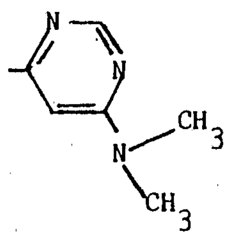 | free base<br>1/2 mole EtOAc<br>hemihydrate<br>187-188° | 57.2<br>(57.0 | 6.7<br>6.7 | 24.2<br>24.2) |
| 10 | 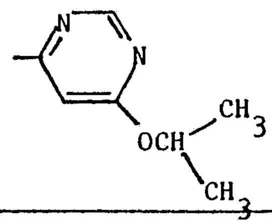 | free base<br>263-265° | 59.3<br>(59.3 | 6.6<br>6.4 | 23.3<br>23.1) |
| 11 | 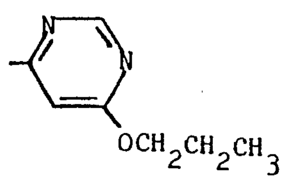 | free base<br>hemihydrate<br>239-241° | 58.5<br>(58.1 | 6.4<br>6.5 | 22.8<br>22.6) |

| Example No. | Het | Form Isolated and m.p. | Analysis % (calculated figures in brackets) C | H | N |
|---|---|---|---|---|---|
| 12 | 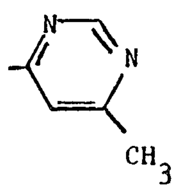 | Dihydrochloride sesquihydrate, 273-276° | 47.3 (47.4 | 5.6 5.9 | 20.5 20.4) |
| 13 | 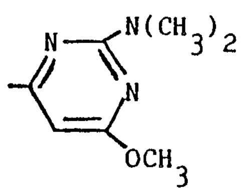 | Dihydrochloride hydrate, 260-261° | 47.2 (47.5 | 5.8 6.1 | 21.4 21.1) |

Method "B"

*Preparation of 4-amino-6,7-dimethoxy-2-[4-(2-phenoxypyrimidin-4-yl) piperazino] quinazoline ¾ hydrate*

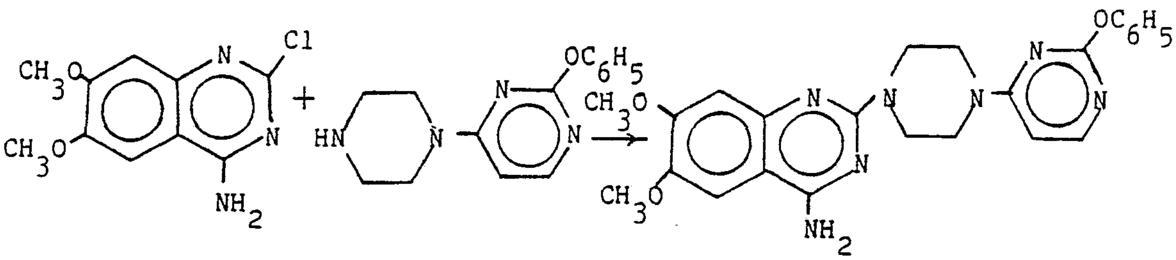

4-Amino-2-chloro-6,7-dimethoxyquinazoline (0.8 g) and 2-phenoxy-4-piperazinopyrimidine dihydrochloride (1.2 g) were heated under reflux in *n*-butanol (50 ml) overnight. The mixture was then evaporated *in vacuo* and the residue partitioned between chloroform/methanol/saturated aqueous sodium carbonate solution (300 ml:100 ml:50 ml). The chloroform/methanol layer was separated, dried ($Na_2SO_4$) evaporated *in vacuo* then the residue (1 g) chromatographed on silica (85 g). The column was eluted with chloroform-methanol (100:0 → 97.5:2.5), appropriate fractions combined, evaporated *in vacuo* and the residue re-crystallized from ethanol to give *4-amino-6,7-dimethoxy-2-[4-2-phenoxypyrimidin-4-yl)piperazino]-quinazoline ¾$H_2O$,* (0.14 g,) m.p. 253—254°.

*Analysis %*

| | | | |
|---|---|---|---|
| Found: | C,60.8; | H,5.5; | N,20.7. |
| Calculated for $C_{24}H_{25}N_7O_3 \cdot \frac{3}{4}H_2O$: | C,60.9; | H, 5.7; | N.20.7. |

Examples 14—20

The following compounds were prepared in the same general manner to "Method B", except that in some cases triethylamine was included in the reaction mixture and other modifications were as follows:

In Example 14, the reaction mixture was evaporated, the residue partitioned between chloroform/water, the solid collected, boiled with methanol, filtered and the filtrate evaporated. The residue was then purified by chromatography. In Example 15 the reaction mixture was evaporated *in vacuo* and the residue crystallized from methanol/diethyl ether. In Example 18, the solid product was collected from the cooled reaction mixture and was re-crystallized from DMF/diethyl ether. In Example 19 the solid product was collected and re-crystallized from DMF. In Example 20 the product from chromatography was converted to the maleate salt using maleic acid which salt was crystallized from methanol.

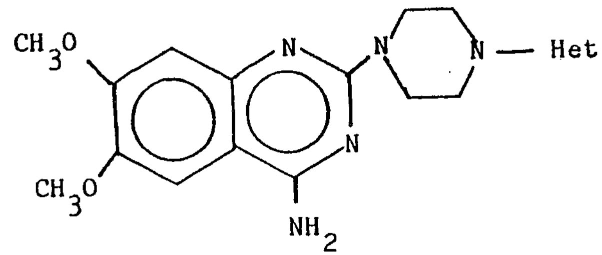

| Example No. | Het | Form Isolated and m.p. | Analysis % (calculated figures in brackets) C | H | N |
|---|---|---|---|---|---|
| 14 | 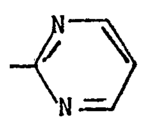 | dihydrochloride 265-266$^{o}$ | 49.0<br>(49.1 | 5.3<br>5.3 | 22.5<br>22.3) |
| 15 | 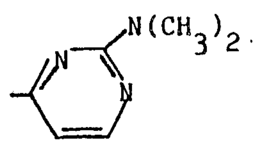 | dihydrochloride dihydrate 255-265$^{o}$ | 46.2<br>(46.2 | 5.8<br>6.2 | 21.8<br>21.6) |

| Example No. | Het | Form Isolated and m.p. | Analysis % (calculated figures in brackets) C | H | N |
|---|---|---|---|---|---|
| 16 | 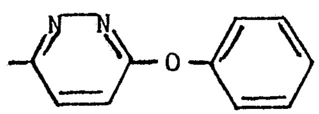 | free base 270-272° | 62.8 (62.7 | 5.6 5.5 | 21.2 21.3) |
| 17 | 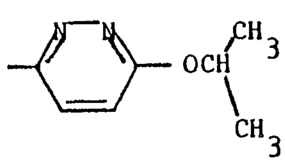 | free base 247-248° | 59.1 (59.3 | 6.6 6.4 | 22.7 23.1) |
| 18 | 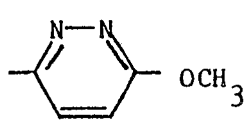 | free base 285-287° | 57.3 (57.4 | 6.0 5.8 | 24.9 24.7) |

| Example No. | Het | Form Isolated and m.p. | Analysis % (calculated figures in brackets) C | H | N |
|---|---|---|---|---|---|
| 19 | 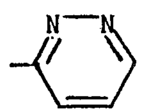 | hydrochloride sesquihydrate 214-215°. | 50.6<br>(50.2 | 5.4<br>5.9 | 22.4<br>22.8) |
| 20 | 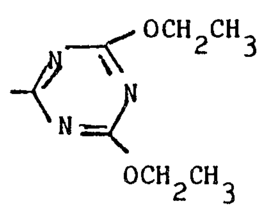 | maleate hydrate 247-248° | 50.6<br>(50.8 | 5.5<br>5.8 | 19.0<br>19.0) |

Example 21

*Preparation of 4-amino-6,7-dimethoxy-2-[4-(2-chloropyrimidin-4-yl)-piperazino]quinazoline*

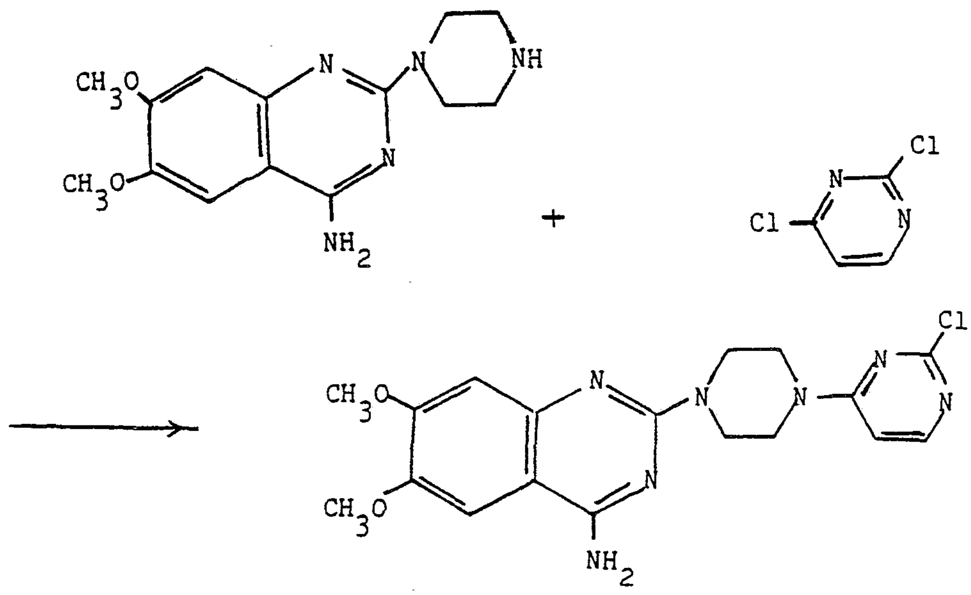

4-Amino-6,7-dimethoxy-2-piperazino-quinazoline (30.0 g), 2,4-dichloropyrimidine (17.3 g) and triethylamine (20.5 g) in ethanol (1200 ml) were heated under reflux for 3 hours. The solid which separated on cooling was filtered off, slurried in hot isopropanol (500 ml), filtered and washed with hot methanol. The product was partitioned between 5% methanol in methylene chloride and 10% aqueous sodium carbonate solution, the organic layer separated, washed with water, dried ($Na_2SO_4$) and evaporated *in vacuo*. The resulting solid was slurried in hot isopropanol, filtered and washed with hot isopropanol to give *4-amino-6,7-dimethoxy-2-[4-(2-chloropyrimidin-4-yl)piperazino]quinazoline* (20 g), m.p. 266°.

*Analysis %:—*

| | | | |
|---|---|---|---|
| Found: | C, 53.75; | H, 5.0; | N, 24.7. |
| Calculated for $C_{18}H_{20}ClN_7O_2$: | C, 53.8; | H, 5.0; | N, 24.4. |

Example 22

*Preparation of 4-Amino-6,7-dimethoxy-2-[4-(2-morpholinopyrimidin-4-yl)-piperazino]quinazoline*

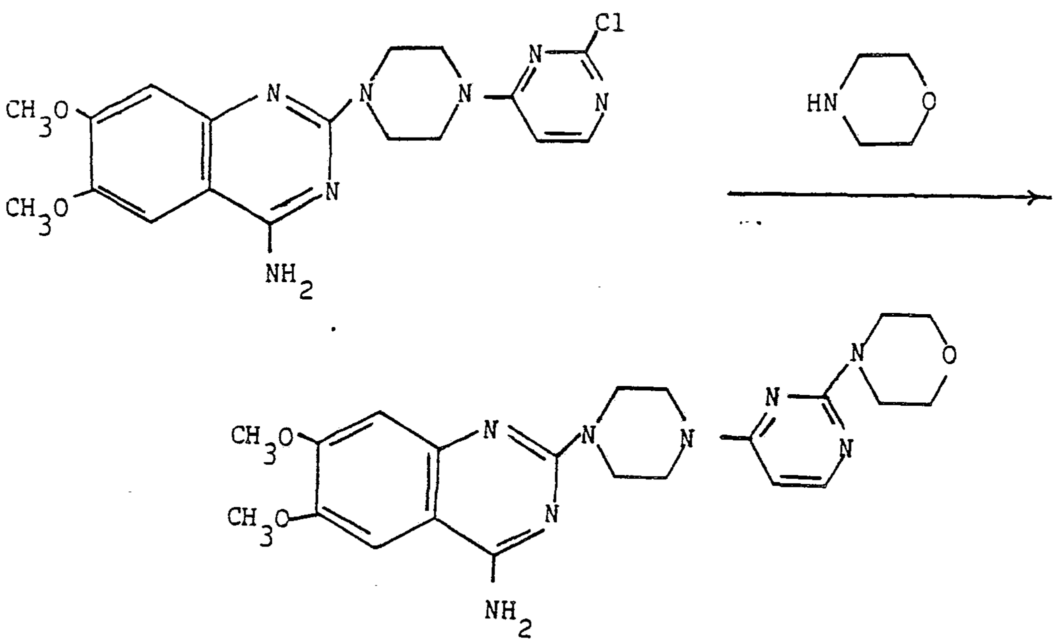

4-Amino-6,7-dimethoxy-2-[4-(2-chloropyrimidin-4-yl)-piperazino]quinazoline (2.0 g) and morpholine (1.1 g) in *n*-butanol (150 ml) were heated in a bomb at 160° for 19 hours. The solvent was evaporated *in*

*vacuo* and the residue partitioned between 5% methanol in chloroform and 5N sodium hydroxide solution. The organic layer was separated, washed with water, dried ($Na_2SO_4$) and evaporated *in vacuo*. The residue was chromatographed on silica (20 g, "Kieselgel" (Trade Mark) 60H) eluting with chloroform. Appropriate fractions were combined and evaporated *in vacuo*. Crystallisation from ethyl acetate gave *4-amino-6,7-dimethoxy-2-[4-(2-morpholinopyrimidin-4-yl)piperazino]quinazoline* (0.8 g) m.p. 232—3°.

*Analysis %:—*

| | | | |
|---|---|---|---|
| Found: | C, 58.9; | H, 6.3; | N, 24.9. |
| Calculated for $C_{22}H_{28}N_8O_3$: | C, 58.4; | H, 6.2; | N, 24.8. |

Example 23

4 - Amino - 6,7 - dimethoxy - 2 - [4 - (2 - {N - cyclopentyl - N - methylamino}pyrimidin - 4 - yl)-piperazino]quinazoline was prepared in a manner similar to that of Example 22, starting from the product of Example 21 and N-cyclopentylmethylamine, but a temperature of 180° for 48 hours was required. The product was characterised as the dihydrochloride dihydrate. m.p. 333—4°.

*Analysis %:—*

| | | | |
|---|---|---|---|
| Found: | C, 50.0; | H, 6.2; | N, 19.8. |
| Calculated for $C_{24}H_{32}N_8O_2.2HCl.2H_2O$: | C, 50.3; | H, 6.7; | N, 19.5. |

Example 24

*4-Amino-6,7-dimethoxy-2-[4-(6-chloro-2-methylpyrimidin-4-yl)-piperazino]quinazoline* was prepared in a manner similar to that of Example 21, starting from the same quinazoline as Example 21 and 4,6-dichloro-2-methyl-pyrimidine. The free base crystallised from dimethylformamide, m.p. 263—264°.

*Analysis %:—*

| | | | |
|---|---|---|---|
| Found: | C, 54.6; | H, 5.55; | N, 23.5. |
| Calculated for $C_{19}H_{22}ClN_7O_2$: | C, 54.9; | H, 5.3; | N, 23.6. |

Example 25

*Preparation of 4-amino-6,7-dimethoxy-2-[4-(pyrimidin-4-yl)piperazino]quinazoline*

4-Amino-6,7-dimethoxy-2-[4-(2-chloro-pyrimidin-4-yl)piperazino]quinazoline (3.1 g) and triethylamine (1.6 g) in dioxan (250 ml) was hydrogenated over Pd on charcoal at 50°/50 psi for 9 hours. The catalyst was filtered off, washed with dioxan, the filtrate and washings combined and evaporated to dryness. The residue was partitioned between chloroform and aqueous sodium hydroxide solution. The organic layer was separated, dried ($Na_2SO_4$) and evaporated *in vacuo*. The residue was chromatographed on silica (32 g, "Kieselgel" {Trade Mark} 60 H) eluting with $CHCl_3$ followed by 0.5% methanol in chloroform. Appropriate fractions were combined, evaporated *in vacuo* and the residue crystallised from methanol to give 4-amino-6,7-dimethoxy 2-[4-(pyrimidin-4-yl)piperazino]quinazoline (0.7 g), m.p. 261°.

*Analysis %:—*

| | | | |
|---|---|---|---|
| Found: | C, 58.65; | H, 5.9; | N, 26.7. |
| Calculated for $C_{18}H_{21}N_7O_2$: | C, 58.8; | H, 5.8; | N, 26.7. |

Example 26

*4-Amino-6,7-dimethoxy-2-[4-(2-methyl-pyrimidin-4-yl)piperazino]quinazoline* was prepared in a manner similar to that of Example 25, starting from 4-amino-6,7-dimethoxy-2-[4-(6-chloro-2-methyl-pyrimidin-4-yl)piperazino]quinazoline. The product crystallized from methanol, m.p. 280—281°.

*Analysis %:—*

| | | | |
|---|---|---|---|
| Found: | C, 59.8; | H, 6.1; | N, 25.8. |
| Calculated for $C_{19}H_{23}N_7O_2$: | C, 59.8; | H, 6.1; | N, 25.7. |

Preparation 1

*Preparation of 2-dimethylamino-4-piperazinopyrimidine, dihydrochloride ¾ hydrate*

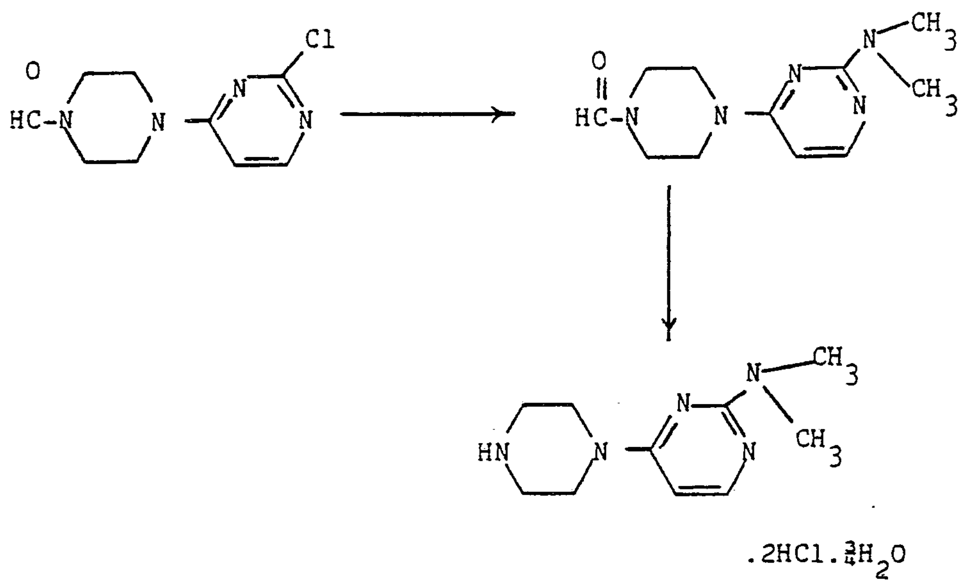

a) 2-Chloro-4-(4-formylpiperazino)pyrimidine (5.0 g) and dimethylamine (7.8 ml, 33% solution in ethanol) (70 ml) were heated under reflux for 8 hours. The solvent was evaporated *in vacuo* and the residue was partitioned between chloroform and water. The aqueous layer was extracted twice with chloroform and the combined chloroform layers dried ($Na_2SO_4$) and evaporated *in vacuo*. The residue was re-crystallized from ethyl acetate to give *2-dimethyl-amino-4-(4-formylpiperazino)pyrimidine* (2.7 g) m.p. 116°.

*Analysis %:—*

| | | | |
|---|---|---|---|
| Found: | C, 55.9; | H, 7.2; | N, 29.5. |
| Calculated for $C_{11}H_{17}N_5O$: | C, 56.1; | H, 7.3; | N, 29.8. |

This product (2.5 g) in methanol (31 ml) and 2N hydrochloric acid (8 ml) was stirred at room temperature for 2¼ hours and then heated on a steam bath for 2¼ hours. The solvent was evaporated *in vacuo* and the residue crystallized from ethanol to give *2-dimethyl-amino-4-piperazinopyrimidine, dihydrochloride, ¾ hydrate* m.p. 260—270°.

*Analysis %:—*

| | | | |
|---|---|---|---|
| Found: | C, 41.0; | H, 6.9; | N, 24.0. |
| Calculated for $C_{10}H_{17}N_5.2HCl.H_2O$: | C, 40.9; | H, 7.9; | N, 23.9. |

Preparation 2

*Preparation of 4-chloro-6-isopropoxy-pyrimidine*

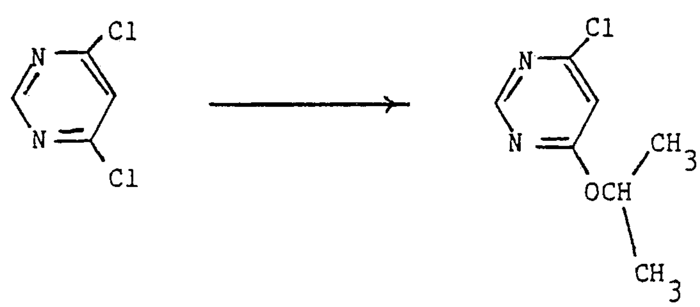

A solution of sodium isopropoxide (prepared from 0.77 g sodium) in isopropanol (230 ml) was added dropwise over 8 hours to a stirred solution of 4,6-dichloropyrimidine (5.0 g) in isopropanol (60 ml) at room temperature. The solvent was evaporated *in vacuo*, the residue taken up in water and extracted three times with diethyl-ether (3 × 70 ml). The combined ether extracts were dried ($Na_2SO_4$) and evaporated *in vacuo* to give *4-chloro-6-isopropoxy pyrimidine* (4.4 g) as an oil, characterized spectroscopically, and used directly.

Preparation 3

*3-Isopropoxy-6-piperazinopyridazine*

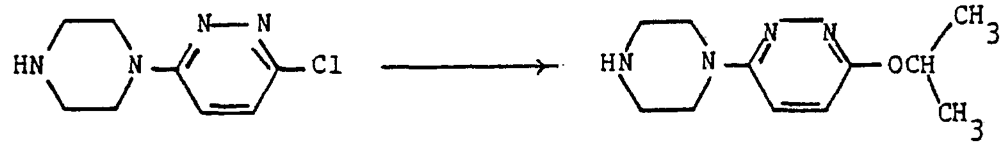

3-Chloro-6-piperazinopyridazine (4.0 g) (J. Med. Chem. 1963, *5*, 541) and sodium isopropoxide, prepared by addition of sodium (0.7 g) to dry isopropanol (70 ml), were heated in a baomb at 130—140° for 10 hours. The solvent was evaporated *in vacuo*, the residue taken up in methylene chloride (300 ml) and the resulting solution washed with water (2 × 50 ml). The organic extract was dried ($Na_2SO_4$) and evaporated *in vacuo* to give *3-isopropoxy-6-piperazinopyridazine* (3.3 g). A sample in ethyl acetate, was converted to the maleate salt by treatment with maleic acid in ethyl acetate. The resulting solid was re-crystallized from ethanol, m.p. 144—145°.

*Analysis %:—*

| | | | |
|---|---|---|---|
| Found: | C, 49.1; | H, 5.7; | N, 11.7. |
| Calculated for $C_{11}H_{18}N_4O.2C_4H_4O_4.\frac{1}{2}H_2O$: | C, 49.2; | H, 5.9; | N, 12.1. |

Preparation 4

*Preparation of 4,6-diethoxy-2-piperazino-1,3,5-triazine*

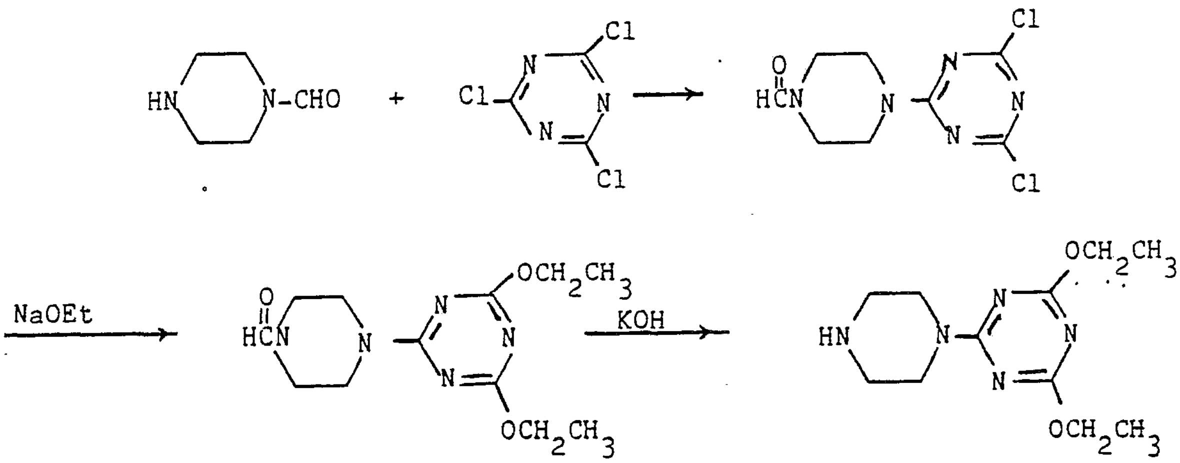


a) *4,6-Dichloro-2-(4-formylpiperazino)-1,3,5-triazine*

1-Formylpiperazine (5.0 g) in dry acetone (28 ml) was added dropwise to a stirred suspension of cyanuric chloride (6.2 g) and sodium bicarbonate (2.58 g) in dry acetone (153 ml) at −35°. The reaction mixture was stirred at −30° for 1 hours. Insoluble material was removed by filtration and washed with acetone. The combined filtrate and washings were evaporated *in vacuo* and the residue taken up in methylene chloride, filtered and the filtrate evaporated *in vacuo*. The resulting solid was re-crystallized twice from ethyl acetate to give in two fractions *4,6-dichloro-2-(4-formylpiperazino)1,3,5-triazine* (3.1 g) m.p. 163—165°.

*Analysis %:—*

| | | | |
|---|---|---|---|
| Found: | C, 36.5; | H, 3.4; | N, 27.1. |
| Calculated for $C_8H_9Cl_2N_5O$: | C, 36.7; | H, 3.5; | N, 26.7. |

b) *4,6-diethoxy-2-(4-formylpiperazino)-1,3,5-triazine*

A solution of sodium ethoxide in dry ethanol (prepared from 1.76 g sodium in 100 ml ethanol) was added dropwise to a stirred suspension of 2,4-dichloro-6-(4-formylpiperazino)-1,3,5-triazine (10 g) in dry ethanol (749 ml). The reaction mixture was stirred at room temperature for 6 hours. The solvent was evaporated *in vacuo* and the residue partitioned between methylene chloride and water. The organic layer was separated, washed 3 times with water, dried ($Na_2SO_4$) and evaporated *in vacuo* to give a solid (7.2 g). Re-crystallization from ethyl acetate gave *4,6-diethoxy-2-(4-formylpiperazino)-1,3,5-triazine* (6.6 g), m.p. 106—108.5°.

*Analysis %:—*

| | | | |
|---|---|---|---|
| Found: | C, 51.3; | H, 6.8; | N, 24.7. |
| Calculated for $C_{12}H_{19}N_5O_3$: | C, 51.2; | H, 6.8; | N, 24.9. |

c) *4,6-Diethoxy-2-piperazino-1,3,5-triazine*

The product from (b) (3.25 g) in potassium hydroxide solution (1N, 20 ml) and ethanol (30 ml) was left at room temperature for 3 hours. Then further potassium hydroxide solution (20 ml) was added. After 45 minutes the reaction mixture was extracted with chloroform (5 × 30 ml), and the combined chloroform extracts dried ($Na_2SO_4$) and evaporated *in vacuo* to give *4,6-diethoxy-2-piperazino-1,3,5-triazine* (3.0 g) as an oil. The product was characterized spectroscopically and used directly without further purification.

Preparation 5

*Preparation of 3-Phenoxy-6-piperazinopyridazine*

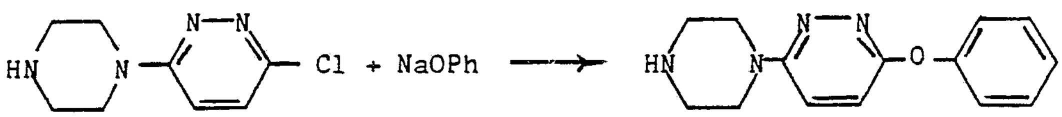

Phenol (39.8 g) was treated with a solution of sodium methoxide in methanol (prepared from 0.7 g sodium in 60 ml dry methanol) and the solvent evaporated *in vacuo* 3-Chloro-6-piperazinopyridazine (4.0 g was added to the resulting mixture of sodium phenate and phenol and the mixture heated at 125—130° for 10 hours with stirring. Methylene chloride (200 ml) was added to the cooled reaction mixture and the solution was washed with aqueous sodium hydroxide solution (3 × 60 ml, 10%). The organic layer was dried ($Na_2SO_4$) and evaporated *in vacuo*. The residue was taken up in isopropanol, treated with charcoal, filtered through "Hyflo" and evaporated *in vacuo*. Chromatography of the residue on silica ("Keiselgel H", 15 g) eluting with chloroform gave *3-phenoxy-6-piperazinopyridazine* (2.0 g). A sample re-crystallized from ethyl acetate as the hemihydrate, m.p. 96—97°.

*Analysis %:—*

| | | | |
|---|---|---|---|
| Found: | C, 63.1; | H, 6.2; | N, 21.4. |
| Calculated for $C_{14}H_{16}N_4O.\frac{1}{2}H_2O$: | C, 63.4; | H, 6.5; | N, 21.1. |

**Claims for the Contracting States: BE CH DE FR GB IT LI LU NL SE**

1. A compound of the formula:—

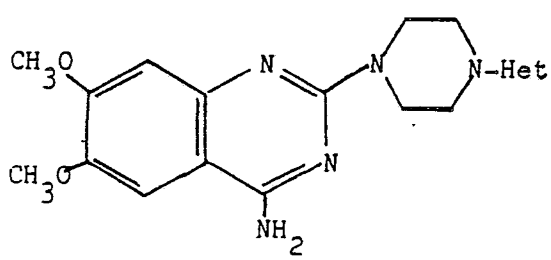

---(I)

or a pharmaceutically acceptable acid addition salt thereof, wherein Het is a nitrogen-containing heterocyclic group selected from:—

(a) 2- or 4-pyrimidinyl unsubstituted or substituted by 1 or 2 substituents each selected from $C_1$—$C_4$ alkyl, $C_1$—$C_4$ alkoxy, halo and $—NR^1R^2$ where $R^1$ is $C_1$—$C_4$ alkyl and $R^2$ is $C_1$—$C_4$ alkyl or $C_3$—$C_7$ cycloalkyl, or $R^1$ and $R^2$ taken together with the nitrogen atom to which they are attached represent morpholino;

(b) 3-pyridazinyl unsubstituted or substituted by 1 substituent selected from $C_1$—$C_4$ alkoxy and phenoxy; and

(c) 1,3,5-triazin-2-yl substituted by 2 substituents selected from $C_1$—$C_4$ alkoxy and

$$\overset{H}{—N}(C_1—C_4\ alkyl).$$

2. A compound as claimed in claim 1 where "Het" is

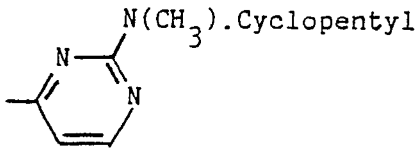

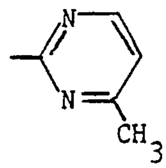 ,

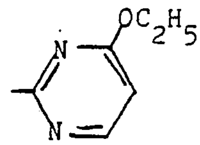 ,

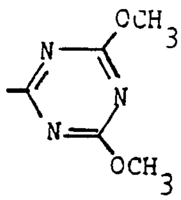 ,

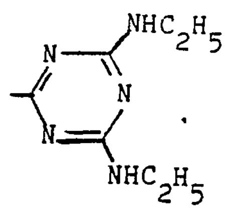 ,

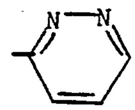 or

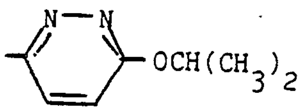

3. A process for preparing a compound of the formula (I) or salt thereof as claimed in claim 1, which comprises reacting a compound of the formula:—

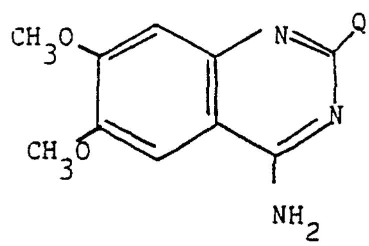

---(III)

wherein Q is a facile leaving group, with a piperazine of the formula:—

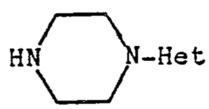

---(IV)

where "Het" is as defined in claim 1, followed by, optionally conversion of the product of the formula (I) into a pharmaceutically acceptable acid addition salt by reaction with a non-toxic acid.

4. A process for preparing a compound of the formula (I) or salt thereof as claimed in claim 1, which comprises reacting a compound of the formula:—

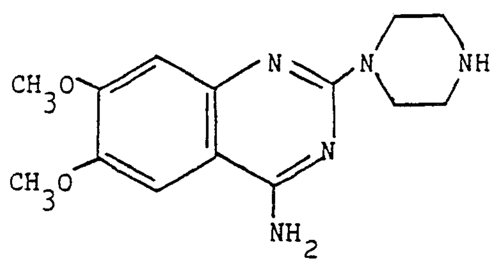

---(V)

with a compound of the formula:—

Q-Het (VI)

wherein Q is a facile leaving group and "Het" is as defined in claim 1, followed by, optionally, conversion of a product of the formula (I) into a pharmaceutically acceptable acid addition salt by reaction with a non-toxic acid.

5. A process as claimed in claim 3 or 4 wherein the reaction of the quinazoline and the heterocycle is carried out in the presence of a tertiary amine base.

6. A process for preparing a compound of the formula (I) as claimed in claim 1 in which "Het" is 2- or 4-pyrimidinyl substituted by —$NR^1R^2$ wherein $R^1$ and $R^2$ are as defined in claim 1, which comprises reacting

the corresponding compound of the formula (I) in which "Het" is substituted by halo with an amine of the formula $R^1R^2NH$.

7. A pharmaceutically composition comprising a compound of the formula (I) as claimed in claim 1 or a pharmaceutically acceptable acid addition salt thereof, together with a pharmaceutically acceptable diluent or carrier.

**Claims for the Contracting State: AT**

1. A process for preparing a compound of the formula:—

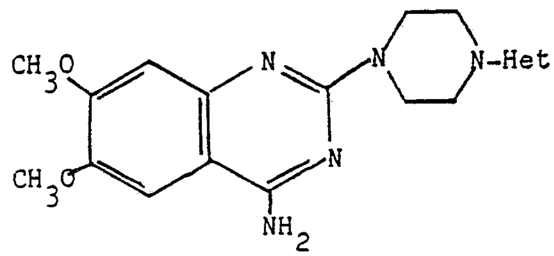

---(I)

or a pharmaceutically acceptable acid addition salt thereof, wherein Het is a nitrogen-containing heterocyclic group selected from:—

(a) 2- or 4-pyrimidinyl unsubstituted or substituted by 1 or 2 substituents each selected from $C_1$—$C_4$ alkyl, $C_1$—$C_4$ alkoxy, halo and —$NR^1R^2$ where $R^1$ is $C_1$—$C_4$ alkyl and $R^2$ is $C_1$—$C_4$ alkyl or $C_3$—$C_7$ cycloalkyl, or $R^1$ and $R^2$ taken together with the nitrogen atom to which they are attached represent morpholino;

(b) 3-pyridazinyl unsubstituted or substituted by 1 substituent selected from $C_1$—$C_4$ alkoxy and phenoxy; and

(c) 1,3,5-triazin-2-yl substituted by 2 substituents selected from $C_1$—$C_4$ alkoxy and

$$-\overset{H}{N}(C_1-C_4\ \text{alkyl}),$$

characterised by reacting a compound of the formula:—

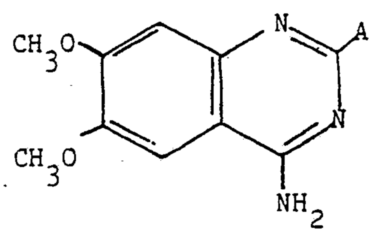

with a compound of the formula:—

B—Het

where "Het" is as defined above, and either A is a facile leaving group and B is

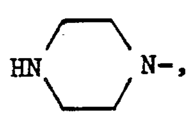

or B is a facile leaving group and A is

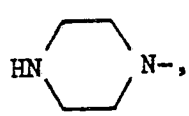

said reaction being followed by, optionally:—

(a) conversion of a compound in which "Het" is substituted by halo into a compound in which "Het" is 2- or 4-pyrimidinyl substituted by —$NR^1R^2$ wherein $R^1$ and $R^2$ are as defined above by reaction with an amine of the formula $R^1R^2NH$; or

(b) reducing a halo substituent on "Het" to H by hydrogenation; or

(c) conversion of a compound of the formula (I) into a pharmaceutically acceptable acid addition salt by reaction with a non-toxic acid.

2. A process as claimed in claim 1, characterised in that said facile leaving group is halo, $C_1$—$C_4$ alkoxy or $C_1$—$C_4$ alkylthio.

3. A process according to any one of the preceding claims characterised in that it is used for the preparation of compounds wherein "Het" is

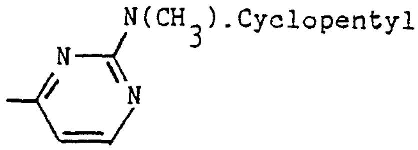
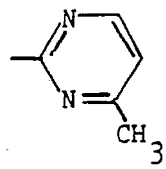
,
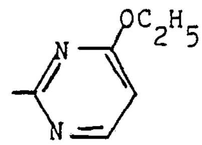
.
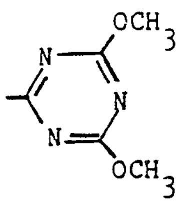
.
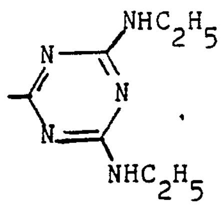
.
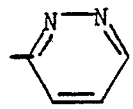
or
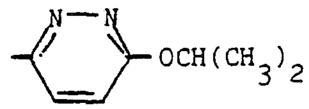
.

**Patentansprüche für die Vertragsstaaten: BE CH DE FR GB IT LI LU NL SE**

1. Verbindung der Formel

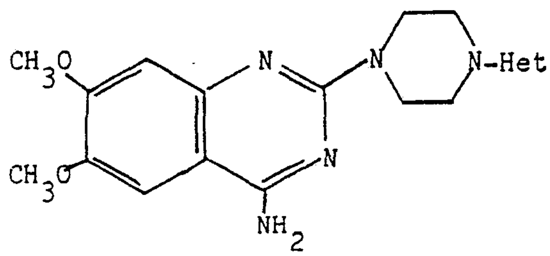
---(I)

oder ein pharmazeutisch annehmbares Säureadditionssalz hiervon, worin Het eine stickstoffhaltige heterocyclische Gruppe ist, ausgewählt unter

a) 2- oder 4-Pyrimidinyl, unsubstituiert oder substituiert durch 1 oder 2 Substituenten, jeweils ausgewählt unter $C_1$—$C_4$-Alkyl, $C_1$—$C_4$-Alkoxy, Halogen und —$NR^1R^2$, worin $R^1$ $C_1$—$C_4$-Alkyl und $R^2$ $C_1$—$C_4$-Alkyl oder $C_3$—$C_7$-Cycloalkyl ist oder $R^1$ und $R^2$ zusammengenommen mit dem Stickstoffatom, an dem sie hängen, Morpholino bedeuten;

b) 3-Pyridazinyl, unsubstituiert oder substituiert durch einen Substituenten, ausgewählt unter $C_1$—$C_4$-Alkoxy und Phenoxy; und

c) 1,3,5-Triazin-2-yl, substituiert durch 2 Substituenten, ausgewählt unter $C_1$—$C_4$-Alkoxy und —NH($C_1$—$C_4$-Alkyl).

2. Verbindung nach Anspruch 1, worin "Het"

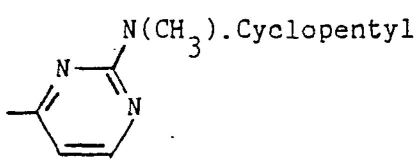
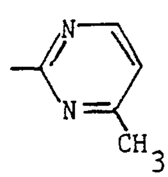
,
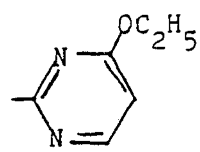
,
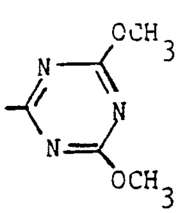
.
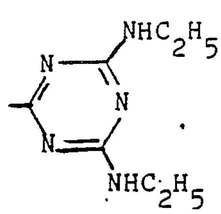
.
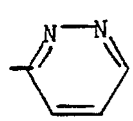
or
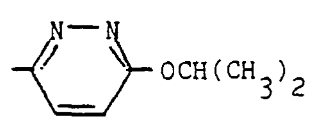
.

ist.

3. Verfahren zur Herstellung einer Verbindung der Formel (I) oder eines Salzes hiervon nach Anspruch 1, das das Umsetzen einer Verbindung der Formel

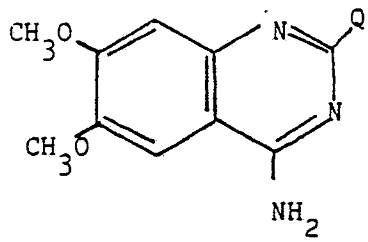

---(III)

worin Q eine leicht austretende Gruppe ist, mit einem Piperazin der Formel

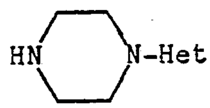

---(IV)

worin "Het" wie in Anspruch 1 definiert ist, gegebenenfalls gefolgt von einer Umwandlung des Produkts der Formel (I) in ein pharmazeutisch annehmbares Säureadditionssalz durch Umsetzen mit einer nicht-toxischen Säure umfaßt.

4. Verfahren zur Herstellung einer Verbindung der Formel (I) oder eines Salzes hiervon nach Anspruch 1, das das Umsetzen einer Verbindung der Formel

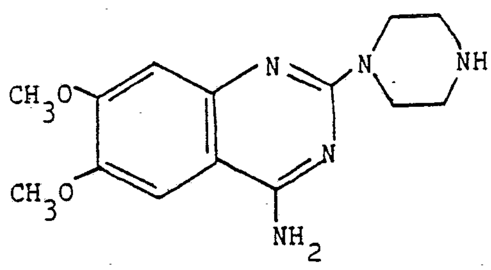

---(V)

mit einer Verbindung der Formel

Q—Het (VI),

worin Q eine leicht austretende Gruppe ist, und "Het" wie in Anspruch 1 definiert ist, gegebenenfalls gefolgt von einer Umwandlung eines Produkts der Formel (I) in ein pharmazeutisch annehmbares Säureadditionssalz durch Umsetzen mit einer nicht-toxischen Säure, umfaßt.

5. Verfahren nach Anspruch 3 oder 4, worin die Umsetzung des Chinazolins und des Heterocyclus in Gegenwart einer tertiären Aminbase durchgeführt wird.

6. Verfahren zur Herstellung einer Verbindung der Formel (I) nach Anspruch 1, worin "Het" 2- oder 4-Pyrimidinyl ist, substituiert durch $—NR^1R^2$, worin $R^1$ und $R^2$ wie in Anspruch 1 definiert sind, das das Umsetzen der entsprechenden Verbindung der Formel (I), worin "Het" durch Halogen substituiert ist, mit einem amin der Formel $R^1R^2NH$ umfaßt.

7. Pharmazeutische Zusammensetzung, umfassend eine Verbindung der Formel (I) nach Anspruch 1 oder ein pharmazeutisch annehmbares Säureadditionssalz hiervon, zusammen mit einem pharmazeutisch annehmbaren Verdünnungsmittel oder Träger.

**Patentansprüche für den Vertragsstaat: AT**

1. Verfahren zur Herstellung einer Verbindung der Formel

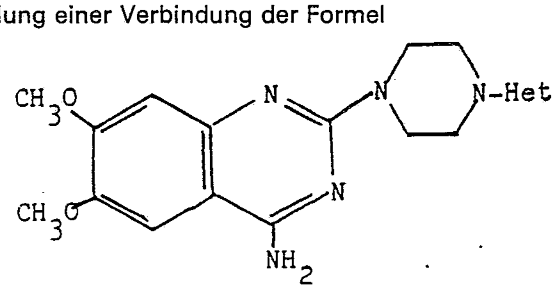

---(I)

oder eines pharmazeutisch annehmbaren Säureadditionssalzes hiervon, worin Het eine stickstoffhaltige heterocyclische Gruppe ist, ausgewählt unter

a) 2- oder 4-Pyrimidinyl, unsubstituiert oder substituiert durch 1 oder 2 Substituenten, jeweils ausgewählt unter $C_1$—$C_4$-Alkyl, $C_1$—$C_4$-Alkoxy, Halogen und —$NR^1R^2$, worin $R^1$ $C_1$—$C_4$-Alkyl und $R^2$ $C_1$—$C_4$-Alkyl oder $C_3$—$C_7$-Cycloalkyl ist oder $R^1$ und $R^2$ zusammengenommen mit dem Stickstoffatom, an dem sie hängen, Morpholino bedeuten;

b) 2-Pyridazinyl, unsubstituiert oder substituiert durch einen Substituenten, ausgewählt unter $C_1$—$C_4$-Alkoxy und Phenoxy; und

c) 1,3,5-Triazin-2-yl, substituiert durch 2 Substituenten, ausgewählt unter $C_1$—$C_4$-Alkoxy und —NH($C_1$—$C_4$-Alkyl), gekennzeichnet durch Umsetzen einer Verbindung der Formel

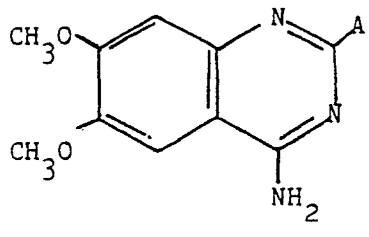

mit einer Verbindung der Formel

B—Het,

worin "Het" wie oben definiert ist, und entweder A eine leicht austretende Gruppe und

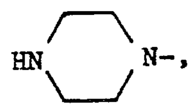

oder B eine leicht austretende Gruppe und A

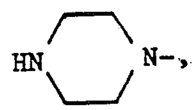

ist, wobei auf die Umsetzung gegebenenfalls

(a) eine Umwandlung einer Verbindung, worin "Het" durch Halogen substituiert ist, in eine Verbindung, worin "Het" 2- oder 4-Pyrimidinyl, substituiert durch —$NR^1R^2$, worin $R^1$ und $R^2$ wie oben definiert sind, ist, durch Umsetzen mit einem Amin der Formel $R^1R^2NH$ oder

(b) eine Reduktion eines Halogen-Substituenten an "Het" zu H durch Hydrieren oder

(c) eine Umwandlung einer Verbindung der Formel (I) in ein pharmazeutisch akzeptables Säureadditionssalz durch Umsetzen mit einer nicht-toxischen Säure folgt.

2. Verfahren nach Anspruch 1, dadurch gekennzeichnet, daß die leicht austretende Gruppe Halogen, $C_1$—$C_4$-Alkoxy oder $C_1$—$C_4$-Alkylthio ist.

3. Verfahren nach irgend einem der vorhergehenden Ansprüche, dadurch gekennzeichnet, daß es zur Herstellung von Verbindungen herangezogen wird, worin "Het"

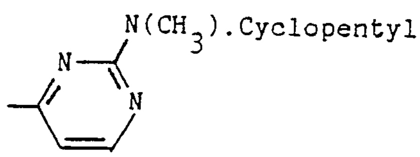

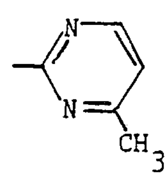 ,

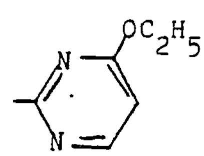 ,

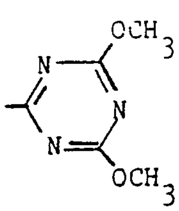 .

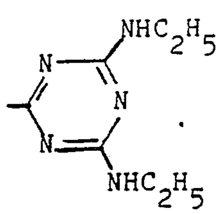 .

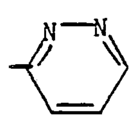 or

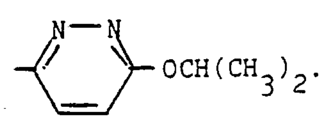

ist.

**Revendications pour les Etats contractants: BE CH DE FR GB IT LI LU NL SE**

1. Un composé de formule:

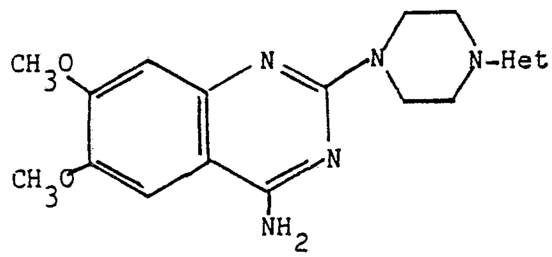

---(I)

ou un sel d'addition d'acide pharmaceutiquement acceptable de ce composé, formule dans laquelle Het est un groupe hétérocyclique contenant de l'azote choisi entre:

(a) un groupe 2- ou 4-pyrimidinyle non substitué ou substitué par 1 ou 2 substituants tous deux choisis entre des substituants alkyle en $C_1$ à $C_4$, alkoxy en $C_1$ à $C_4$, halogéno et —$NR^1R^2$ où $R^1$ est un groupe alkyle en $C_1$ à $C_4$ et $R^2$ est un groupe alkyle en $C_1$ à $C_4$ ou un groupe cycloalkyle en $C_3$ à $C_7$, ou bien $R^1$ et $R^2$ représentent un groupe morpholino conjointement avec l'atome d'azote avec lequel ils sont liés;

(b) un groupe 3-pyridazinyle non substitué ou substitué par 1 substituant choisi entre des groupes alkoxy en $C_1$ à $C_4$ et phénoxy; et

(c) un groupe 1,3,5-triazin-2-yle substitué par 2 substituants choisis entre des groupes alkoxy en $C_1$ à $C_4$ et

H
—N(alkyle en $C_1$ à $C_4$).

2. Composé suivant la revendication 1, dans lequel "Het" est un groupe

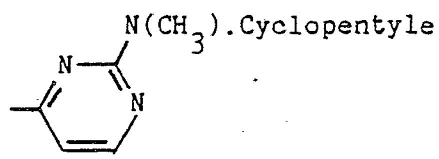 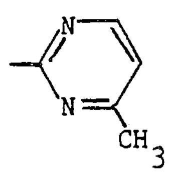, 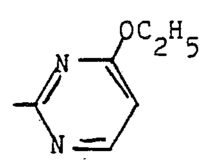,

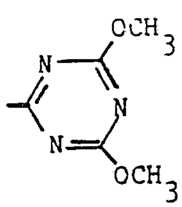, 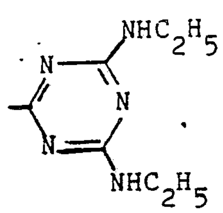, 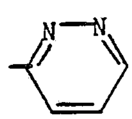 or 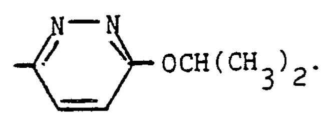.

3. Procédé de préparation d'un composé de formule (I) ou d'un sel de ce composé suivant la revendication 1, qui consiste à faire réagir un composé de formule:

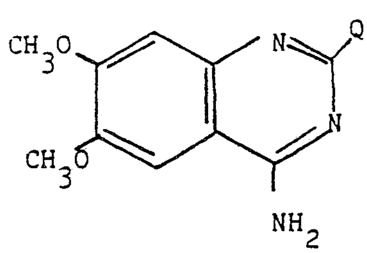

---(III)

dans laquelle Q est un groupe aisément partant, avec une pipérazine de formule

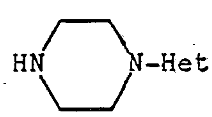

---(IV)

dans laquelle "Het" est tel que défini dans la revendication 1, la réaction étant suivie éventuellement de la transformation du produit de formule (I) en un sel d'addition d'acide pharmaceutiquement acceptable par réaction avec un acide non toxique.

4. Procédé de préparation d'un composé de formule (I) ou d'un sel de ce composé suivant la revendication 1, qui consiste à faire réagir un composé de formule:

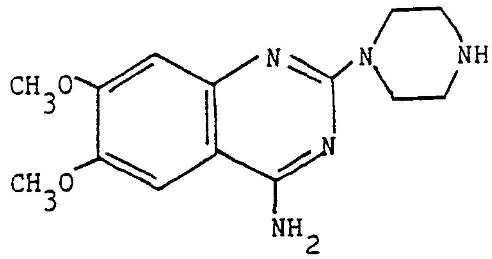

---(V)

avec un composé de formule:

Q—Het (VI)

dans laquelle Q est un groupe aisément partant et "Het" est tel que défini dans la revendication 1, après quoi, le cas échéant, on transforme un produit de formule (I) en un sel d'addition d'acide pharmaceutiquement acceptable par réaction avec un acide non toxique.

5. Procédé suivant la revendication 3 ou 4. dans lequel la réaction de la quinazoline et de l'hétérocycle est conduite en présence d'une base aminée tertiaire.

6. Procédé de préparation d'un composé de formule (I) suivant la revendication 1, dans laquelle "Het" est un groupe 2- ou 4-pyrimidinyle substitué par un substituant —$NR^1R^2$ dans lequel $R^1$ et $R^2$ sont tels que définis dans la revendication 1, qui consiste à faire réagir le composé correspondant de formule (I) dans laquelle "Het" est substitué par un substituant halogéno avec une amine de formule $R^1R^2NH$.

7. Composition pharmaceutique comprenant un composé de formule (I) suivant la revendication 1 ou un sel d'addition d'acide pharmaceutiquement acceptable de ce composé en association avec un diluant ou support acceptable du point de vue pharmaceutique.

**Revendications pour l'Etat contractant: AT**

1. Procédé de préparation d'un composé de formule:

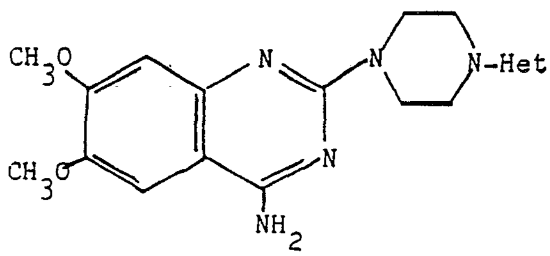

---(I)

ou d'un sel d'addition d'acide pharmaceutiquement acceptable de ce composé, formule dans laquelle Het est un groupe hétérocyclique contenant de l'azote choisi entre:

(a) un groupe 2- ou 4-pyrimidinyle non substitué ou substitué par 1 ou 2 substituants tous deux choisis entre des substituants alkyle en $C_1$ à $C_4$, alkoxy en $C_1$ à $C_4$, halogéno et —$NR^1R^2$ où $R^1$ est un groupe alkyle en $C_1$ à $C_4$ et $R^2$ est un groupe alkyle en $C_1$ à $C_4$ ou un groupe cycloalkyle en $C_3$ à $C_7$, ou bien $R^1$ et $R^2$ représentent un groupe morpholino conjointement avec l'atome d'azote avec lequel ils sont liés;

(b) un groupe 3-pyridazinyle non substitué ou substitué par 1 substituant choisi entre des groupes alkoxy en $C_1$ à $C_4$ et phénoxy; et

(c) un groupe 1,3,5-triazin-2-yle substitué par 2 substituants choisis entre des groupes alkoxy en $C_1$ à $C_4$ et

H
—N(alkyle en $C_1$ à $C_4$),

caractérisé par la réaction d'un composé de formule:

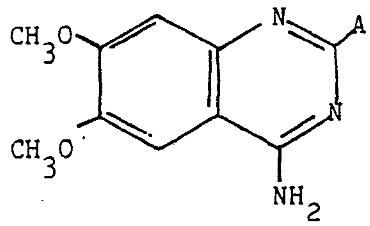

avec un composé de formule:

B—Het

dans laquelle 'Het" est tel que défini ci-dessus, et A est un groupe aisément partant et B est un groupe

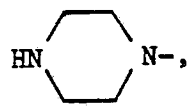

ou bien B est un groupe aisément partant et A est un groupe

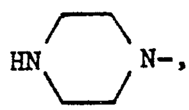

ladite réaction étant suivie, le cas échéant:

(a) de la transformation d'un composé dans lequel "Het" est substitué par un radical halogéno en un composé dans lequel "Het" est un groupe 2- ou 4-pyrimidinyle substitué par un substituant —$NR^1R^2$, dans lequel $R^1$ et $R^2$ sont tels que défini ci-dessus, par réaction avec une amine de formule $R^1R^2NH$; ou

(b) la réduction d'un substituant halogéno porté par "Het" en H par hydrogénation; ou

(c) la transformation d'un composé de formule (I) en un sel d'addition d'acide pharmaceutiquement acceptable par réaction avec un acide non toxique.

2. Procédé suivant la revendication 1, caractérisé en ce que ledit groupe aisément partant est un groupe halogéno, alkoxy en $C_1$ à $C_4$ ou alkylthio en $C_1$ à $C_4$.

3. Procédé suivant l'une quelconque des revendications précédentes, caractérisé en ce qu'il est utilisé pour la préparation de composés dans lesquels "Het" représente

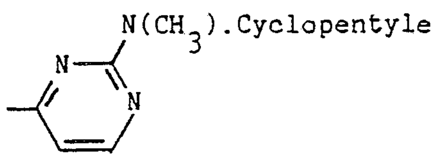

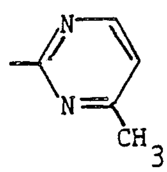 ,

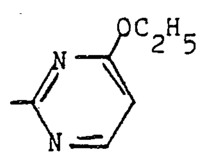 ,

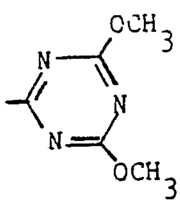 ,

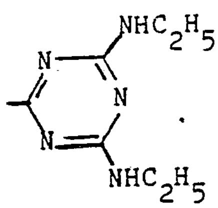 ,

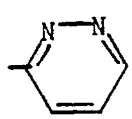 or

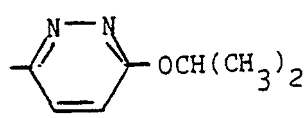 .